Europäisches Patentamt

European Patent Office

Office européen des brevets

(19)

⊙ Numéro de publication: **0 272 174**
**A2**

(12)

# DEMANDE DE BREVET EUROPEEN

(21) Numéro de dépôt: **87402592.7**

(22) Date de dépôt: **17.11.87**

(51) Int. Cl.⁴ **C12N 9/88** , A61K 39/10 , A61K 39/07 , A61K 37/56 , A61K 39/40

(30) Priorité: **17.11.86 FR 8615963**

(43) Date de publication de la demande:
**22.06.88 Bulletin 88/25**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15(FR)**

Demandeur: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Alonson, Jean-Michel**
**51 rue de Cornouailles**
**F-78180 Montigny le Bretonneux(FR)**
Inventeur: **Brezin, Colette**
**11 bis avenue Emile Deschanel**
**F-75007 Paris(FR)**
Inventeur: **Crenon, Isabelle**
**7 rue Alexis Bouvier**
**F-92700 Colombes(FR)**
Inventeur: **Djavadi, Lisa**
**50 avenue Duquesne**
**F-75007 Paris(FR)**
Inventeur: **Guiso-Maclouf, Nicole**
**13 bld Saint-Marcel**
**F-75013 Paris(FR)**
Inventeur: **Ladant, Daniel**
**24 rue Ampère**
**F-94230 Cachan(FR)**

(74) Mandataire: **Peaucelle, Chantal et al**
**S.C. Ernest Gutmann - Yves Plasseraud 67, boulevard Haussmann**
**F-75008 Paris(FR)**

(54) **Préparations d'adenyl cyclase purifiées, procédé d'obtention de ces préparations et leurs applications biologiques.**

(57) Les préparations d'adényl cyclase de l'invention possèdent une pureté élevée et sont pratiquement totalement dépourvues de produits contaminants bactériens.

## PREPARATIONS D'ADENYL CYCLASE PURIFIEES, PROCEDE D'OBTENTION DE CES PREPARATIONS ET LEURS APPLICATIONS BIOLOGIQUES :

L'invention a pour objet des préparations d'adényl cyclase purifiées, un procédé d'obtention de ces préparations et leurs applications biologiques.

On sait qu'une adényl cyclase, secrétée et activée par la calmoduline, ou en abréviation AC, est exprimée par des bactéries du genre Bordetella ou Bacillus anthracis , responsables d'infections aiguës chez les vertébrés.

Divers travaux ont porté sur l'étude des effets biologiques de cet enzyme sur les cellules des vertébrés.

Cependant, l'étude de ses caractéristiques de structure et de ses propriétés se trouve limitée par le caractère hétérogènedes préparations d'AC décrites à ce jour.

La mise au point par les inventeurs d'un procédé de purification d'AC a permis d'obtenir des préparations d'AC ayant une activité spécifique au moins deux fois supérieure à celles des préparations connues et de mettre en évidence des caractéristiques propres de l'AC. L'obtention de préparations de pureté élevée a permis également de développer des applications biologiques de grand intérêt pour le diagnostic, le traitement et/ou la prévention des maladies provoquées par les bactéries évoquées ci-dessus.

L'invention a donc pour but de fournir de nouvelles préparations d'AC de pureté élevée.

Elle vise également à fournir un procédé d'obtention de ces préparations de mise en oeuvre aisée.

L'invention vise, en outre, les applications biologiques de ces préparations purifiées. En particulier, elle vise à fournir des anticorps polyclonaux formés contre ces préparations et selon un aspect spécialement intéressant des préparations d'anticorps monoclonal reconnaissant spécifiquement un épitope de l'AC et exerçant un rôle protecteur vis-à-vis des infections provoquées par les Bordetella.

L'invention vise en outre les réactifs biologiques élaborés à partir des anticorps polyclonaux et plus spécialement monoclonaux. Les applications biologiques comprennent également l'élaboration de vaccins contre les maladies infectieuses provoquées par ces bactéries, par mise en jeu de l'épitope reconnu spécifiquement par les préparations d'anticorps monoclonal.

Les préparations enzymatiques d'AC selon l'invention sont caractérisées en ce qu'elles possèdent une pureté élevée et sont pratiquement totalement dépourvues de produits contaminants bactériens, notamment de pertussis toxines, de lipopolysaccharide (ou LPS) et d'hémagglutinine filamenteuse (ou FHA).

Ces préparations sont également caractérisées en ce que l'AC se présente sous une forme homogène sédimentant sur un gradient de densité en saccharose avec un coefficient S égal à 3,6.

Dans ces préparations homogènes, l'AC peut exister sous deux formes moléculaires d'environ 45 et 43 kDa respectivement, structurellement apparentées.

Selon un autre aspect, ces préparations sont caractérisées en ce qu'elles possèdent une activité pouvant atteindre et même dépasser 1600 µmole de cAMP min-1 mg-1. On notera qu'une unité d'AC correspond à une µmole d'adénosine 3':5'-monophosphate ou, en abréviation, cAMP, formé en 1 min à 30°C à pH 8.

Dans les préparations définies ci-dessus, l'AC est libre ou sous forme d'un complexe en association avec des protéines eucaryotes, plus spécialement avec la calmoduline.

La constante d'association de l'AC avec la calmoduline dans ces complexes, est de 10 nM environ.

Les préparations d'AC selon l'invention sont également caractérisées en ce que l'activité catalytique est portée par un fragment de 25 kDa, site de combinaison à la calmoduline et protégé par celle-ci contre les digestions trypsiques.

Les préparations d'AC de pureté élevée selon l'invention sont des préparations élaborées à partir de cultures de bactéries exprimant l'AC. Il s'agit plus spécialement des bactéries pathogènes dont l'AC est capable d'interférer avec l'AC des cellules eucaryotes. On citera plus particulièrement celles du genre Bordetella ou Bacillus anthracis.

Des préparations d'AC de Bordetella particulièrement préférées au regard des applications thérapeutiques envisagées comprennent de l'AC provenant de Bordetella responsables de pathologies respiratoires des vertébrés telles que, chez l'homme, Bordetella pertussis et Bordetella parapertussis, et chez l'animal, Bordetella bronchiseptica et Bordetella avium.

L'invention vise également un procédé d'obtention de préparations d'adényl cyclase de haute pureté.

Ce procédé est caractérisé par l'étape de mise en contact d'un surnageant préalablement concentré de cultures de bactéries exprimant l'adényl cyclase, ou d'un extrait de ces bactéries, avec de la calmoduline.

Selon un mode de réalisation permettant d'obtenir l'enzyme pur sous forme libre, on utilise de la calmoduline fixée à un support. L'enzyme présent dans le surnageant concentré ou dans l'extrait bactérien, fixé à la calmoduline, lors de l'étape de mise en contact, est ensuite récupéré à l'aide d'un agent dénaturant, qui est à son tour éliminé pour obtenir la préparation enzymatique libre.

Le support auquel est fixée la calmoduline est constitué par un matériau inerte vis-à-vis de la préparation renfermant l'enzyme, et capable de retenir les molécules de poids moléculaire élevé.

Dans une variante, le support est constitué par un gel.

On utilise avantageusement un produit sur lequel est déjà fixée la calmoduline, tel que le produit commercial Affigel calmoduline®.

Pour obtenir la fixation de l'AC contenue dans le surnageant concentré ou dans l'extrait bactérien, on soumet le mélange gel-calmoduline et surnageant ou extrait à une agitation à basse température.

Selon une autre variante, le support sur lequel est fixée la calmoduline est un matériau filtrant du type des filtres Millipores®, capable de retenir sélectivement au moins la majeure partie de l'enzyme.

D'une manière générale, le matériau filtrant est en nitrocellulose ou en une matière plastique et présente une porosité de 0,45-0,22 μm.

On récupère l'adényl cyclase fixée sur le complexe support-calmoduline à l'aide d'un agent dénaturant. De préférence, on utilise de l'urée en milieu tampon. Une dissolution satisfaisante de l'AC dans l'urée est obtenue avec de l'urée 4 à 8,8M. L'urée est ensuite éliminée de la préparation enzymatique par exemple par dialyse ou filtration.

On obtient ainsi des préparations enzymatiques de pureté élevée, ayant une activité spécifique atteignant ou même dépassant 1100 u/mg de protéines.

On notera que lorsqu'on utilise un surnageant concentré, on purifie l'adénylate extracytoplasmique. Avec un extrait bactérien, le procédé de l'invention conduit à l'obtention de préparation d'AC periplasmique.

Les mêmes caractéristiques sont observées pour l'AC d'origine extracellulaire ou periplasmique.

Selon un autre mode de réalisation permettant d'obtenir l'enzyme sous forme d'un complexe de grande stabilité avec la calmoduline, on ajoute de la calmoduline à une préparation enrichie d'au moins 90 % en adényl cyclase, puis on met en contact le mélange, de préférence en réalisant une chromatographie d'affinité, avec un échangeur d'ions de faible porosité, tel que le DEAE-Sephacel®. En augmentant la force ionique du tampon de lavage, on élue le complexe.

Cette variante permet d'obtenir avec des rendements d'environ 40% des préparations d'AC fixée à la calmoduline, de grande stabilité, dont l'activité spécifique peut atteindre et même dépasser 1600 u. mg de protéine.

La préparation enrichie en adényl cyclase mise en oeuvre est obtenue à partir d'un surnageant de culture concentré, dont le pH a été amené à 7,0 et filtré à l'aide d'un échangeur d'ions, avantageusement du DEAE-Sephacel®.

Le surnageant concentré mis en oeuvre dans le procédé de l'invention est obtenu avantageusement en soumettant un surnageant de cultures bactériennes exprimant l'AC à une ou plusieurs opérations de filtrations à l'aide de filtres de nitrocellulose ou en matière plastique, de porosité avantageusement de 0,45-0,22 μm, en particulier ceux de type Millipores®, puis en incubant les filtres avec un agent détergent afin de libérer l'AC et en éliminant de la préparation d'AC les matéraux insolubles présents.

L'agent détergent est un détergent non ionique, de préférence du Triton® ou du Np40®.

L'extrait bactérien utilisable aux fins de purification de l'adényl cyclase est obtenu, par exemple, par traitement des cellules bactériennes exprimant l'adényl cyclase avec de l'urée et récupération du surnageant.

Les bactéries exprimant l'adényl cyclase utilisées pour les cultures sont choisies parmi les bactéries pathogènes dont l'AC est capable d'interférer avec l'AC des cellules eucaryotes. Il s'agit plus particulièrement de Bordetella ou Bacillus anthracis.

Parmi les Bordetella, on citera : Bordetella pertussis, Bordetella bronchiseptica, Bordetella parapertusis et Bordetella avium.

L'étude sur un modèle de souris, d'infections produites par des bactéries synthétisant de l'adényl cyclase avec des rendements élevés, a permis de mettre en évidence le rôle protecteur d'anticorps polyclonaux ou encore d'anticorps monoclonaux formés contre les préparations enzymatiques purifiées définies ci-dessus.

L'invention concerne donc également en tant que produits nouveaux, des anticorps polyclonaux dirigés contre ces préparations enzymatiques.

Ces anticorps sont obtenus par exemple par immunisation d'animaux tels que le lapin ou la souris à l'aide des préparations d'AC en question dans lesquelles l'AC est libre ou sous forme de complexe avec des protéines eucaryotes, notamment la calmoduline, et récupération selon les techniques classiques des immunsérums renfermant les anticorps, puis des anticorps eux-mêmes.

L'invention concerne également des anticorps monoclonaux tels qu'obtenus par fusion, à un myélome de souris, de lymphocytes spléniques de souris immunisées avec l'adényl cyclase purifiée.

Ces préparations d'immunoglobulines monoclonales sont caractérisées en ce qu'elles reconnaissent spécifiquement un épitope de l'AC purifiée qui permet l'induction de la synthèse d'anticorps protecteurs.

L'invention vise particulièrement la préparation d'anticorps monoclonaux (8-25) qui immunoprécipite, dans les extraits bruts, et les préparations de l'enzyme purifiée à partir des cellules bactériennes, un triplet de 50,45 et 43kDa ainsi qu'une protéine de haut poids moléculaire supérieur à 100 kDa.

La souche d'hybridome productrice d'anticorps monoclonaux entre également dans le cadre de l'invention.

Une telle souche a été déposée à la Collection Nationale de Cultures de Microorganismes sous le n° I-610 le 9 octobre 1986.

D'une manière avantageuse, les anticorps selon l'invention exercent un effet immunoprotecteur vis-à-vis de l'AC. Ces propriétés sont mises à profit pour protéger l'homme ou l'animal des effets pathogènes des bactéries ci-dessus. Dans cette application, on a recours à une administration locale, dans le cas d'infections respiratoires par voie intranasale ou intratachéale, ou par voie parentérale. On utilise les excipients classiques tels que le phosphate de calcium ou l'hydroxyde d'aluminium. Les anticorps monoclonaux de l'invention permettent d'effectuer une sérothérapie et une séroprophylaxie spécifiques.

Les anticorps de l'invention sont également utilisables pour la purification de l'AC, y compris des autres espèces en cas de réactivité croisée et pour le diagnostic précoce de la maladie. Ils sont en effet particulièrement appropriés pour le dépistage des malades et des porteurs latents.

Ils permettent également l'isolement de bactéries et de mutants ce qui leur confère un intérêt supplémentaire.

Ces applications en tant que réactifs de diagnostic mettant en jeu des radioimmunoessais. le test ELISA, ou des déterminations par produits marqués par $^{125}$I sont des produits nouveaux qui entrent dans le champ de l'invention.

Selon un autre aspect de grand intérêt, l'invention fournit également, grâce aux anticorps monoclonaux définis ci-dessus, un épitope actif de l'AC reconnu spécifiquement. Les essais de toxicologie réalisés sur l'AC purifiée ayant démontré l'absence de toxicité de cette enzyme administrée par voie intranasale sur la souris, cette AC purifiée est avantageusement utilisée pour l'élaboration de principes vaccinants, sous réserve qu'elle ne donne pas de réactions croisées avec l'AC des eucaryotes traités.

Les vaccins selon l'invention sont donc caractérisés en ce qu'il s'agit de vaccins moléculaires renfermant un épitope actif de l'AC en association avec un véhicule pharmaceutique. capable d'induire une infection et des effets toxiques chez l'homme ou l'animal. On a recours avantageusement aux doses et formes d'administration habituelles.

D'autres caractéristiques et avantages de l'invention apparaîtront dans les exemples qui suivent. relatifs à l'obtention de préparations purifiées d'AC et à leurs applications biologiques, et en se référant aux figures, qui représentent respectivement :

    -les figures 1A à 1C, la variation de la radioactivité et de l'activité AC, pour différentes dilutions de surnageants de culture concentrés, ces surnageants ayant été incubés avec de la $^{125}$I calmoduline, puis centrifugés selon un gradient de sucrose ;

    -les figures 2a et 2b, les photos de préparations purifiées selon l'invention soumises à une analyse SDS-PAGE ;

    -la figure 3, une carte peptidique par protéolyse limitée de $^{125}$I-AC :

    -les figures 4a et 4b, la variation de l'activité en pourcentage en fonction du temps :

    -les figures 5a et 5b, l'analyse en gradient de sucrose de produits de digestion par la trypsine d'un complexe $^{125}$IAC-calmoduline ;

    -la figure 6, un radioimmunoessai d'AC ;

    -la figure 7, les cinétiques de l'infection pulmonaire induite par des inoculats ;

    -la figure 8, une photo d'une alvéolite hémorragique chez la souris produite par des clones de B. pertussis ;

    -les figures 9a et 9b, deux photos correspondant respectivement à une analyse selon SDS-PAGE de surnageant concentré, de deux préparations d'AC purifiées et d'un extrait bactérien, et à un immunoblot avec l'anticorps monoclonal 8-25.

Dans les exemples qui suivent. l'ioduration des produits est réalisée comme suit.

On opère à température ambiante en utilisant la méthode à la chloramine-T (Greenwood et al.. Biochem. J. 89. 114-123, 1963).

La calmoduline iodurée a une radioactivité spécifique de 20 Ci minol : elle est stable sur deux mois lorsqu'on la stocke à -20°C.

Avant l'ioduration, le complexe AC-calmoduline obtenu après purification sur DEAE-Sephacel. est chromatographié sur hydroxylapatite afin d'éliminer l'agent détergent. La préparation $^{125}$I-AC est stable

pendant deux mois à -20°C.

Les analyses des produits des exemples sont effectuées comme suit.

L'activité de l'AC est mesurée selon la méthode de White A.A. dans Methods Enzymol., 38C, 41-46, 1974 telle que modifiée par Hanoune et al. (J. Biol. Chem., 252, 2 039-2 046, 1977).

La réaction est effectuée dans un milieu contenant 50 mM Tris-HCl, pH 8, 6 mM de $MgCl_2$, 100 $\mu$g d'albumine de sérum bovin, 0,12 mM de $CaCl_2$ et 0,1 $\mu$M de calmoduline lorsqu'elle est ajoutée.

Une unité d'AC correspond, comme indiqué ci-dessus, à 1 $\mu$mole de cAMP formée en une minute à 30°C à pH 8. Les protéines sont mesurées soit selon Bradford (Anal. Biochem., 248-254) ou selon Schulz et al. (Anal. Biochem., 91, 354-356, 1978).

On effectue la méthode SDS-PAGE selon Laemli (Nature, Londres, 227, 680-685, 1976). Les gels sont colorés à l'argent comme décrit par Morrissey (Anal. Biochem., 117, 307-310, 1981) ou autoradiographiés après exposition aux pellicules X-omat AT à -70°C.

EXEMPLE 1 : Culture de <u>Bordetella</u> <u>pertussis</u> et obtention d'un surnageant à concentration élevée en adényl cyclase.

On effectue une préculture de Bordetella pertussis, 18323, phase I (souche ATCC 9797) et d'un variant avirulent, phase IV, spontanément, stable de cette souche, pendant 48 heures à 36° C, sur un milieu d'agar modifié Stainer-Scholte supplementé avec de la cyclodextrine (selon Imaizumi et al. dans J. Chim. Microbiol., 17, 781-786, 1983). On transfère ensuite la préculture dans un milieu liquide modifié de Stainer-Scholte sans cyclodextrine.

Les cultures liquides sont soumises à agitation à 150 t/min. pendant 24 heures à 36° C, dans un erlenmeyer de 1 litre contenant 250 ml de milieu. On poursuit la culture jusqu'à l'obtention d'une densité optique $DO_{650}$ = 1,2 ± 0,2. Les bactéries sont alors éliminées par centrifugation à 5000 $^x$g durant 25 min.

Les surnageants de culture sont conservés à -30°C jusqu'à utilisation ou concentrés directement.

Aux fins de concentration, on filtre, sur des filtres de type Millipore® HAWP de 0,45 $\mu$m, 3 litres de surnageant de culture contenant environ 10 $\mu$g de protéines et 0,1 unité d'enzyme par ml. On retient ainsi plus de 90% de l'activité enzymatique sur les filtres. 80% environ de cette activité peuvent être récupérés par incubation des filtres dans 40 ml d'un tampon A constitué par du Tris.HCl, 50 mM, pH 8 contenant 6 mM de $MgCl_2$ et 0,1% de Triton X.100®. Le matériau insoluble est éliminé par centrifugation durant 30 min. à 15000 $^x$ g à 4° C. L'activité spécifique du surnageant de culture concentré est de 115 unités par milligramme de protéine.

EXEMPLE 2 : Procédé d'obtention d'adényl cyclase extracytoplasmique purifiée sous forme libre.

On ajoute 40 ml de surnageant de culture concentré à 0,8 à 1 ml d'Affigel-calmoduline® et on agite lentement le mélange à 4° C pendant 18 heures. Plus des 2/3 de l'activité enzymatique sont retenus sur le gel. On fait sédimenter l'Affigel-calmoduline par centrifugation à 300 $^x$ g pendant 1 minute, on lave à plusieurs reprises avec NaCl 0,5 M dans le tampon A. On récupère l'adényl cyclase à partir du gel à l'aide de 2,5 ml d'urée 8,8 M dans le tampon A. On élimine l'urée par filtration sur une colonne de Séphadex G-25®, équilibrée avec le tampon A.

Cette préparation enzymatique possède une activité spécifique de 1100 unités/mg de protéine. On peut la conserver à -80° C sans perte d'activité sur plusieurs semaines.

EXEMPLE 3 : Procédé d'obtention d'adényl cyclase purifiée à partir d'extraits bactériens.

On soumet une culture de bactéries exprimant de l'AC activable par de la calmoduline à un traitement avec de l'urée 8M durant environ 2 heures à température ambiante. On récupère le surnageant qui constitue l'extrait bactérien et on le met en contact avec le gel Affigel-calmoduline dans les conditions rapportées ci-dessus.

On obtient une préparation enzymatique de pureté élevée présentant sensiblement la même activité spécifique.

EXEMPLE 4 : Procédé d'obtention d'AC purifiée sous forme d'un complexe avec la calmoduline.

On utilise un surnageant de culture concentré. On ajuste ce surnageant à pH 7.0 à l'aide d'imidazole 1,5M (pH 6,5) et on fait passer 40 ml de ce matériau sur une colonne de 5 ml de DEAE-Sephacel. équilibrée avec le tampon A à un débit de 10 ml h. On ajoute de la calmoduline dans l'effluent (0.2 $\mu$M concentration finale). On fait passer le mélange sur une colonne de 3 ml de DEAE-Sephacel. On fixe ainsi 95% de l'activité de AC sur la colonne. Après lavage avec NaCl 0.1M dans le tampon A. on élue le complexe AC-calmoduline avec NaCl 0.3M dans le tampon A, à un débit de 3 ml h.

L'activité spécifique de l'AC est de 1600 u mg de protéines.

Cet enzyme peut être conservé à -80° C sans perte de son activité sur plusieurs semaines.

EXEMPLE 5 : Etude des préparations enzymatiques d'AC purifiées.

-Etude des propriétés de liaison à la calmoduline :

On fait incuber différentes quantités d'enzyme avec une quantité constante de ($I^{125}$) calmoduline. puis on sépare le mélange par centrifugation selon un gradient de densité en saccharose.

Cette opération est réalisée comme suit :

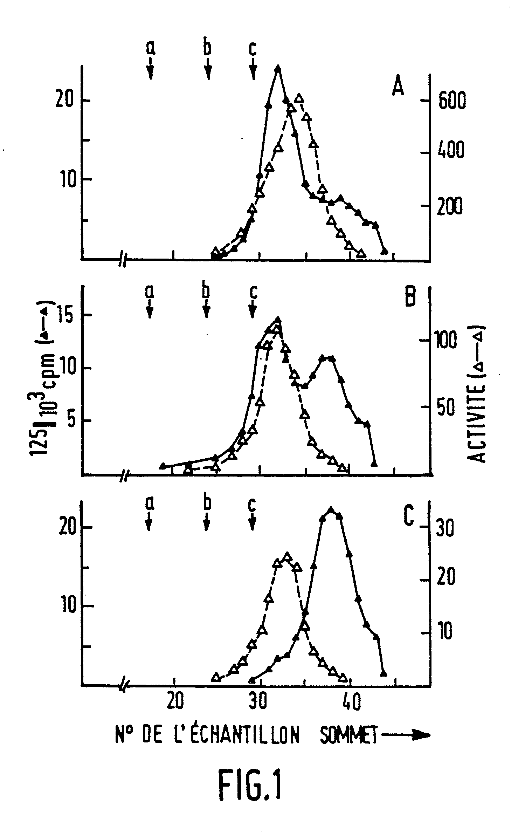

On fait incuber 0,5 ml du tampon A contenant 20 nM de ($^{125}I$) calmoduline de cerveau de boeuf (20 Ci/mmol) et 3 dilutions de surnageant de culture concentré (A : 5 u ml, B : 1 u ml. C : 0,2 u ml). L'incubation est réalisée à 4° C durant une heure. On effectue ensuite une centrifugation avec gradient en saccharose de 5 à 20% durant 16 heures à 35000 t/min. dans un rotor SW41®. On recueille des fractions de 0.5 ml dont on analyse la radioactivité et l'activité AC (mesurée en présence de calmoduline et exprimée en $10^3$ u/ml). Pour chaque opération, on utilise comme référence, a : la pyruvate kinase ($S_{20.w}$ = 10S) b : la lactate déhydrogénase ($S_{20,w}$ = 7S); c : la créatine kinase ($S_{20,w}$ = 5S). Les résultats obtenus sont rapportés sur les figures 1A à 1C sur lesquelles on indique d'une part la radioactivité (courbe ▲ —— ▲) et d'autre part l'activité AC (courbe Δ —— Δ) pour les fractions recueillies. Les courbes A. B, C correspondent aux dilutions des surnageants indiquées ci-dessus).

L'examen de ces figures montre que lorsqu'on utilise de l'AC en excès par rapport à la calmoduline radiomarquée (figure 1A) 80% de la radioactivité sont déplacés d'un coefficient de sédimentation de 2S correspondant à celui de la calmoduline à 3,9S. L'enzyme mesurée par son activité. sédimente sous forme d'un pic non symétrique ayant une valeur S de 3,6 qui correspond à celle de l'AC libre et d'un épaulement à 3,9 S. Cet épaulement correspond au complexe AC-calmoduline.

Lorsque le rapport de l'AC à la calmoduline est de 1:1 (figure 1B) ou 1:5 (figure 1C) on récupère respectivement 70% de la radioactivité dans le pic 3,9S. La proportion de ($^{125}I$) calmoduline libre à celle liée dans les trois conditions, permet d'estimer une constante d'association avec l'AC de 10nM. Cette valeur est très proche de celle calculée de 5 nM à partir de la courbe d'activation de l'activité de l'AC par la calmoduline.

-Etude par la méthode SDS-PAGE (sodium dodecylsulfate-polyacrylamid gel electrophoresis)

On étudie selon la méthode SDS-PAGE (10% d'acrylamide) avec coloration du gel à l'aide d'argent. l'AC purifiée avec a) de l'Affigel-calmoduline, ou b) du DEAE-Sephacel.

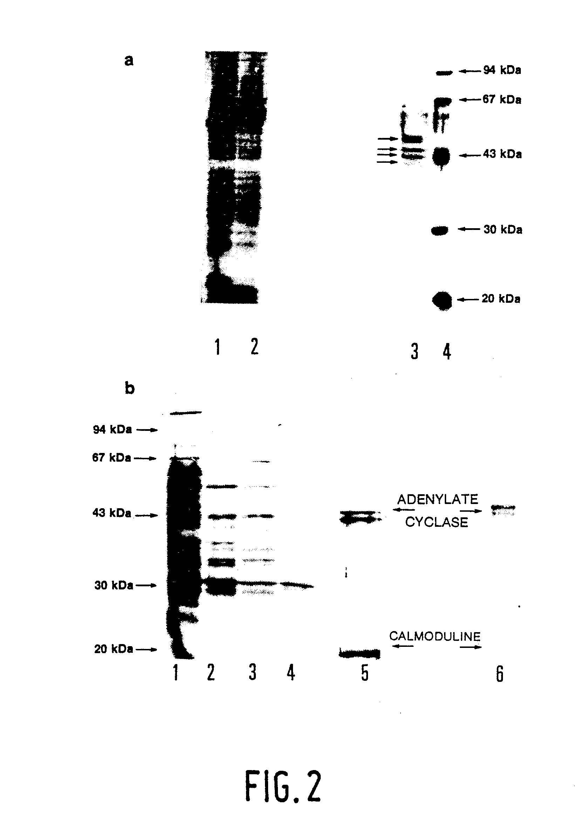

Les résultats obtenus sont rapportés sur les figures 2a et 2b. Sur la figure 2a, la piste 1 correspond au surnageant de culture concentré, la piste 2 aux protéines non liées à l'Affigel-calmoduline et la piste 3 aux protéines éluées à partir de l'Affigel-calmoduline.

Sur la figure 2b. la piste 1 correspond au surnageant de culture concentré, la piste 2 aux protéines non liées à la première colonne de DEAE-Séphacel, la piste 3 aux protéines non liées à la 2ème colonne de DEAE-Séphacel, la piste 4 aux protéines éluées durant le lavage du DEAE-Séphacel. la piste 5 aux protéines éluées à partir de la 2ème colonne de DEAE-Séphacel et la piste 6 à la préparation de ($^{125}I$) AC purifiée sur DEAE-Séphacel. Comme on le constate à l'examen de la figure 2a, piste 3. la préparation d'enzyme purifié renferme quatre protéines de 50kDa, 45 kDa. 43kDa, et 40kDa. Les bandes supplémentaires observées sont dues à des artefacts de coloration de l'argent.

L'examen de la figure 2b, montre qu'une préparation purifiée à l'aide de DEAE-Séphacel renferme 2 polypeptides de poids moléculaire respectif de 45000 et 43000 et de la calmoduline en excès. Après traitement par l'iode, les deux polypeptides et la calmoduline sont marqués (figure 2b, piste 6).

-Etablissement d'une carte des peptides par protéolyse limitée de la ($^{125}$I) AC.

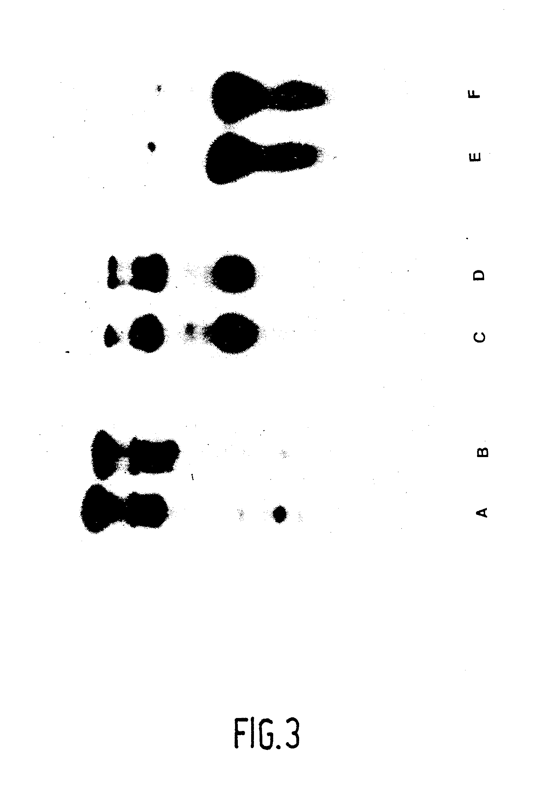

Les 2 polypeptides marqués de 45kDa et 43kDa sont prélevés d'un gel SDS (5.5% d'acrylamide) sur lequel on applique un complexe ($^{125}$I) d'AC-calmoduline. On effectue une autoradiographie du gel pour visualiser les positions des 2 polypeptides et les coupes de gel sont appliquées sur un deuxième gel SDS (15% d'acrylamide) en présence d'une protéase de Staphylococcus aureus(protéase V$_3$) comme décrit par Cleveland et al., J. Biol. Chem., 262, 1 102-1 106, 1977). On rapporte sur la figure 3 les résultats obtenus : A, C et E correspondent aux profils de digestion de peptides de 45kDa et B. D et F aux profils de digestion de la protéine de 43kDa avec respectivement 0,075 µg, 0,75 µg et 7,5 µg de protéase.

Comme le montre cette figure, les deux polypeptides présentent des profils de digestion protéolytique semblables, ce qui indiquent qu'ils sont structurellement apparentés.

-Etude de la sensibilité à la digestion par la trypsine de l'AC libre et de l'AC liée à la calmoduline.

On étudie les différences conformationnelles éventuelles entre l'AC libre et l'AC liée à la calmoduline en investigant leur sensibilité à la digestion par la trypsine.

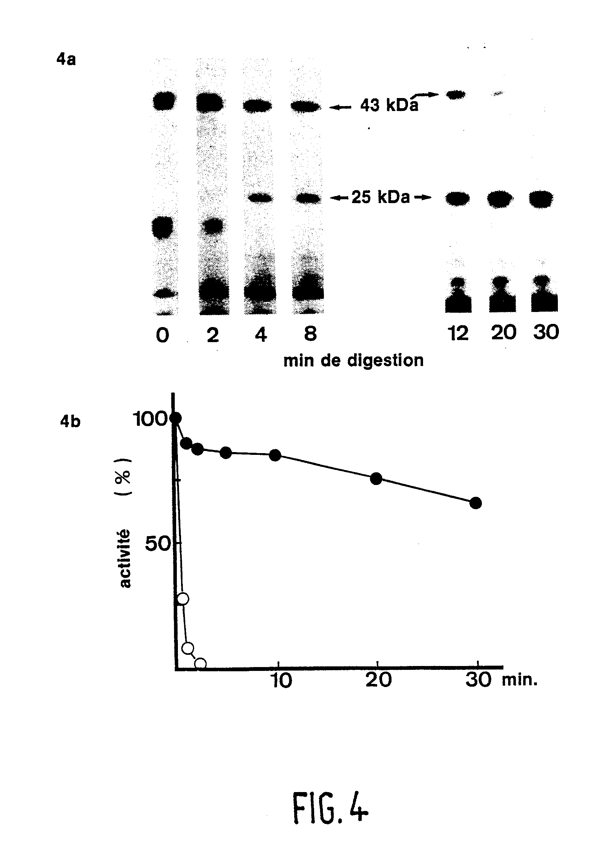

Les résultats obtenus sont rapportés sur les figures 4a et 4b.

Les expériences dont les résultats sont rapportés sur la figure 4a sont réalisées comme suit :

On incube à 50°C avec 50 µl de trypsine TPCK (un microgramme par ml dans le tampon A) 50 µl d'AC purifiée sur Affigel-calmoduline (O-O) ou d'un complexe AC-calmoduline purifiée sur DEAE-Séphacel (●-●) (1,5 u/ml 1 µg/ml dans le tampon A). A des temps déterminés, on dilue des parties aliquotes de 10 µl dans 20 µl d'inhibiteur de trypsine de soja (0,2 mg/ml dans le tampon A), on mesure l'activité en présence de calmoduline.

Les expériences dont les résultats sont rapportés sur la figure 4b sont réalisées comme suit :

On traite un complexe ($^{125}$I) AC-calmoduline (1,5 u/ml) dans les conditions évoquées ci-dessus. Des parties aliquotes sont déterminées soit pour leur activité AC ou examinées selon la méthode SDS-PAGE (10% d'acrylamide).

Les résultats obtenus montrent que l'incubation de l'AC libre avec la trypsine à 0° C (1:1. p:p) entraîne une inactivation complète de l'enzyme dans les 2 minutes (voir figure 4b). Dans les mêmes conditions. le complexe de l'AC-calmoduline apparaît beaucoup plus résistant à l'inactivation protéolytique. 70% de l'activité enzymatique est maintenue après 30 min. d'incubation.

Le SDS-PAGE des produits du complexe AC-calmoduline marquée par ($^{125}$I) montre une conversion rapide du polypeptide de 45kDa en polypeptide 43kDa, qui est lui-même clivé progressivement en peptide de 25kDa. Cette dernière forme apparaît beaucoup plus résistante à la protéolyse ultérieure.

La calmoduline marquée par ($^{125}$I) en excés par rapport au complexe ($^{125}$I)enzyme-calmoduline est rapidement dégradée par la trypsine comme le montre la figure 4a.

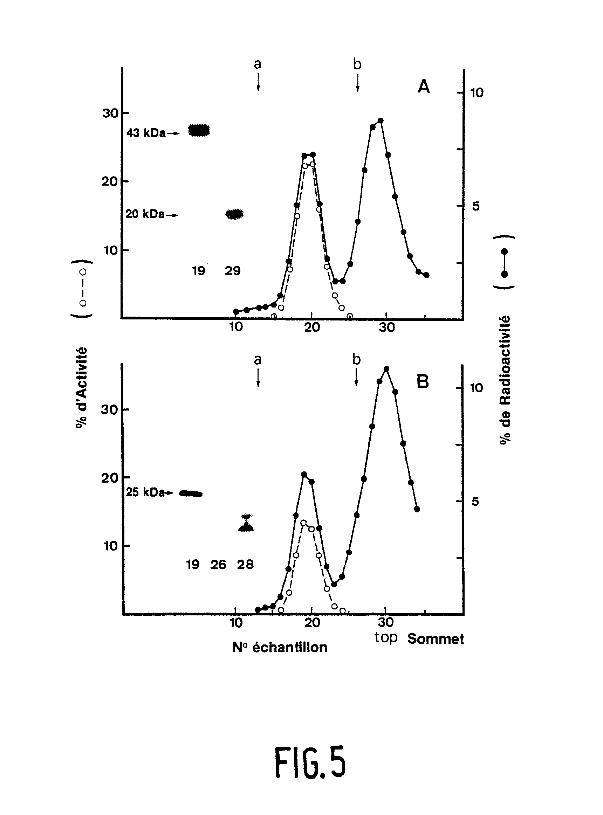

Pour étudier la fonction catalytique du peptide de 25kDa, on a analysé sur un gradient de saccharose les produits de digestion par la trypsine du complexe($^{125}$I) AC-calmoduline. On rapporte sur les figures 5A et 5B les résultats obtenus en opérant comme suit :

Après 16 heures de centrifugation à 52000 t/min. dans un rotor SW60® à 4° C. sur des gradients de saccharose allant de 5 à 20%, on recueille des fractions de 120 µl dont on mesure l'activité AC (en présence de calmoduline) et la radioactivité. Dans les parties insérées sur les courbes. on indique l'autoradiographie selon une analyse SDS-PAGE (10% d'acrylamide) des fractions indiquées.

La figure 5A correspond au complexe non traité d'$^{125}$I AC-calmoduline ($12 \times 10^3$ ; $6,8 \times 10^5$ cpm)et la figure 5B correspond au complexe ($^{125}$I)AC-calmoduline digéré par de la trypsine pendant 30 mn à 0° C (l'activité initiale en l'absence de trypsine est de $22 \times 10^3$ u; $1,15 \times 10^5$ cpm. Après 30 minutes de digestion : $11 \times 10^3$ u; $1,15 \times 10^5$ cpm. Les résultats sont exprimés en pourcentage de l'activité initiale. Les références sont, a : la créatine kinase ($S_{20,w} = 5S$); b : adénylate kinase ($S_{20,w} = 2,4S$).

On constate que dans l'expérience témoin. sans traitement à la trypsine, la radioactivité est retrouvée dans 2 pics distincts correspondants au complexe enzymatiquement actif et à la calmoduline libre (figure 5a).

Après 30 minutes d'incubation en présence de trypsine. 55% de l'activité originale est récupérée. L'analyse de l'enzyme digéré par la trypsine montre qu'elle a la même valeur S apparente que l'enzyme non traité (figure 5b). Cependant, l'analyse des fractions correspondant au pic d'activité sur le gel SDS révèle seulement un polypeptide de 25kDa. Il apparaît ainsi que. bien que la trypsine clive les liaisons peptidiques de la molécule d'AC, les fragments tiennent encore ensemble par des interactions non convalentes selon la même conformation active que dans le complexe non digéré d'AC-calmoduline.

EXEMPLE 6 : Etude radio-immunologique de l'AC purifiée

On utilise le complexe d'AC-calmoduline pour former des anticorps anti-enzymes chez la souris.

On utilise des souris femelles BALB/c de 4 semaines qu'on immunise par voie sous-cutanée à 3 reprises à une semaine d'intervalle avec 10 µl de préparation d'AC purifiée sur Affigel-calmoduline. On effectue un rappel 1 mois plus tard en utilisant une préparation d'AC purifiée à l'aide de DEAE-Séphacel. On recueille les sérums 3 jours après le rappel.

Les anticorps anti-AC sont détectés en utilisant le radio-immunoessai réalisé comme indiqué ci-après. Toutes les réactions immunologiques sont effectuées dans le tampon A. le mélange réactionnel contient 100 µl de ($^{125}$I)AC (2 × 10$^4$ cpm et 4 × 10$^4$ u d'activité enzymatique), 100 µl d'immunsérum de souris anti-AC dilué et 100 µl d'AC non marquée ou de surnageant de culture concentré. Après 18 heures d'incubation à 4° C, on ajoute 100 µl d'anticorps de lapin, anti-immunoglobuline de souris et 100 µl de polyéthylène glycol à 8%, dilué dans un sérum de souris préimmun. Après avoir effectué une autre incubation de 30 min. à 4° C, on centrifuge les tubes à 2000 × g pendant 30 min., on élimine les surnageants et la radioactivité du précipité est mesurée dans un compteur gamma.

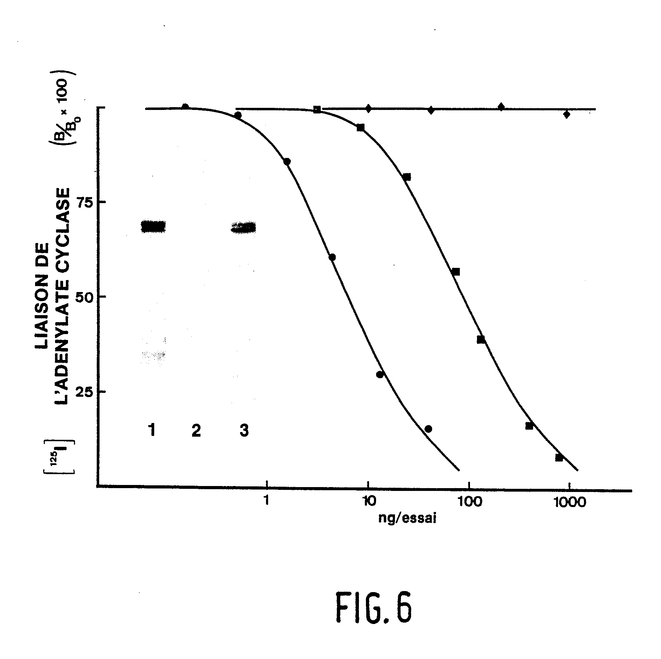

Les résultats du radioimmunoessai sont rapportés sur la figure 6.

Sur cette figure Bo correspond à la fixation initiale de 50% de ($^{125}$I) AC liée à l'antisérum en l'absence d'AC non marquée et B au pourcentage de ($^{125}$I) AC résiduelle, liée en présence de différentes quantités d'AC non marquées ( ♦ -♦) ou de surnageant de culture concentré de la souche 18323 ( . - . ) ou d'un surnageant de culture du variant phase IV avirulent (■-■) les parties insérées correspondent aux résultats obtenus avec une préparation ($^{125}$I)AC incubée avec un sérum préimmun ou un antisérum anti-AC de souris 18 heures à 4°C puis les complexes d'anticorps ($^{125}$I) AC sont précipités comme décrits ci-dessus. Le précipité est solubilisé dans un tampon et étudié selon la méthode SDS-PAGE (10% d'acrylamide). La piste 1 représente la préparation témoin de ($^{125}$I) AC, la piste 2 l'immunoprécipitation avec le sérum avant immunisation et la piste 3 l'immunoprécipitation avec l'antisérum de souris anti-AC.

Comme le montre la figure 6, l'antisérum de souris précipite spécifiquement l'enzyme marquée par ($^{125}$I) et non la calmoduline marqué par ($^{125}$I).

En développant un radioimmunoessai pour déterminer la concentration d'AC dans les surnageants bruts ou les extraits bactériens de Bordetella pertussis, on obtient les résultats suivants :

Une courbe dose-réponse typique est donnée sur la figure 6 et ert obtenue avec une dilution de sérum anti-AC de 1:2000. En utilisant ce radioimmunoessai, il est possible de détecter une quantité aussi faible que 5 ng. La courbe d'inhibition obtenue avec le surnageant concentré est parallèle à la courbe de référence déterminée avec l'enzyme purifié. Aucune AC ne peut être détectée dans le surnageant de culture dépourvu d'activité enzymatique, obtenu à partir du dérivé phase IV avirulent de la souche. On en conclut que le variant phase IV produit des niveaux d'AC qui ne peuvent être décelés et ne produit pas de protéines actives.

EXEMPLE 7 : Etude de l'inhibition de l'activité d'AC par l'antisérum anti-AC.

On incube 300 µl d'AC (60×10$^3$ u/ml) purifiée sur Affigel-calmoduline en l'absence ou en présence de 250 µg d'albumine de sérum bovin ou de 50 µl de sérum. Après 18 heures à 0° C, les parties aliquotes sont prélevées et on détermine l'activité AC en présence de calmoduline. Les complexes antigène-anticorps sont précipités comme décrits ci-dessus et l'activité enzymatique est déterminée dans le surnageant. Les résultats obtenus sont rapportés dans le tableau 1 ci-après :

TABLEAU 1

| Additions | Activité adényl cyclase | $(10^{-3}$ u/ml) |
| --- | --- | --- |
| | avant précipitation | après précipitation |
| - | 62 | 62 |
| Albumine de sérum bovin | 100 | 64,5 |
| Sérum préimmun | 92 | 66 |
| Sérum anti-adénylate cyclase | 9 | 0,7 |

On constate que l'incubation d'AC avec de la sérumalbumine ou du sérum préimmun, entraîne une augmentation de 30 à 40% de l'activité, tandis que l'incubation de l'enzyme avec un antisérum spécifique conduit à une inhibition de 90% de l'activité. De plus, les anticorps anti-AC précipitent 99% de l'enzyme actif étant donné que moins de 1% de l'activité initiale se trouve dans le surnageant.

EXEMPLE 8 : Etude des effets protecteurs d'anticorps anti-AC.

On cultive la souche Bordetella pertussis 18323 sur agar Bordet Gengou, supplémentée avec 15% (v/v) de sang de cheval défibriné (BGB) à 36°C pendant 72 heures. On effectue une sub-culture d'une colonie hémolytique (hly +) dans une infusion Steiner-Scholte pendant 20 à 24 heures jusqu'à l'obtention de A₆₅₀ de 1,0 qui correspond à des unités formant des colonies (CFU) de 2 (Δ 1) ×10⁹ ml-1. 50 µl de cette culture sont injectés dans les orifices externes des narines de souris femelles Swiss de 3 semaines. A partir d'une culture de Bordetella pertussis sur BGB à partir d'homogéneisats de poumons de souris infectés 48 h, on sélectionne les colonies au regard de la stabilité du phenotype hly +, en effectuant plusieurs essais. il apparaît que la fréquence d'obtention des variants hly-est approximativement de 10³. Deux clones isogéniques sont isolés : 1 clone hly + désigné ci-après VIIIA7 et un deuxième clone, hly-, désigné 7-1. Le clone VIIIA7 et la souche parentale sont positifs vis-à-vis des effets du facteur engendrant la leucocytose (LPF) et les effets d'agglomération sur les cellules d'ovaires de hamsters chinois (CHO) du Ptx, les effets d'hémaglutination du FHA et la production d'AC extra cellulaire. Le clone 7-1 est négatif vis-à-vis de l'expression de Ptx et de AC mais retient l'activité FHA.

La souche parentale et ses deux dérivés isogéniques cultivés sur un milieu agar Stainer-Scholte supplémenté avec de la cyclodextrine, sont ensuite cultivés dans des sub-cultures de bouillon de milieu Stainer-Scholte pour injection aux souris.

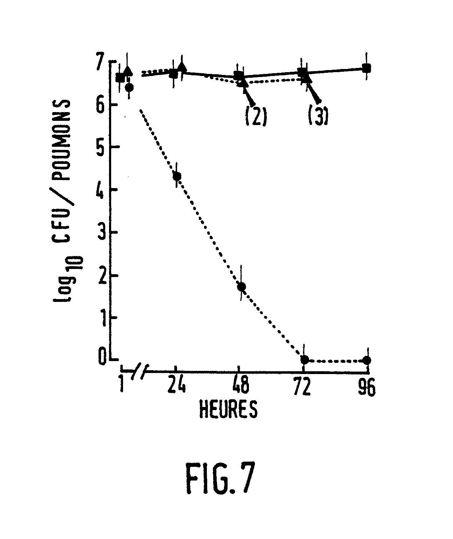

Pour estimer la virulence (en termes de quantités de CFU capable de s'établir et de se répliquer dans les poumons), et la pathogénicité (en termes de capacité à induire des lésions tissulaires) de la souche parentale 18323 et de ses dérivés isogéniques VIIIA7 et 7-1, on utilise un modèle murin quantitatif décrit par Alonso J. M. et al. dans FEMS Microbiol. Letter, sous presse. Sur la figure 7, on rapporte les cinétiques de l'infection pulmonaire induite par les 3 inoculats : la souche parentale et le clone VIIIA7, expriment une virulence équivalente tandis que le clone 7-1 est éliminé des poumons dans les 3 jours.

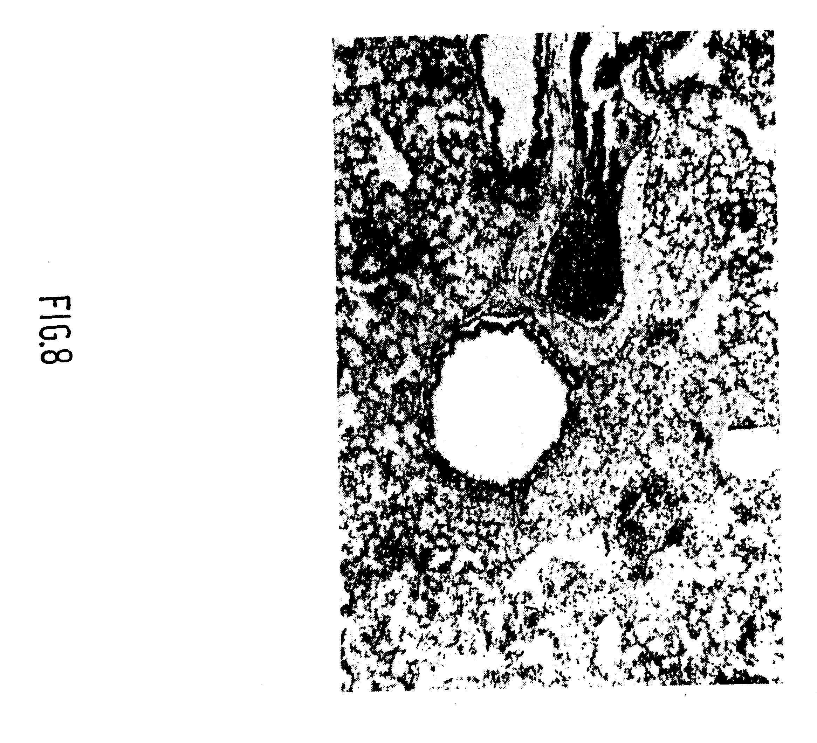

Bien que l'intensité de l'infection induite apparaisse équivalente à celle de la souche parentale, le clone VIIIA7 est léthal pour la souris en produisant une alvéolite hémorragique complète (figure 8). Compte tenu de ce résultat, on a cherché à identifier le facteur responsable de la léthalité et à distinguer le clone pathogène VIIIA7 de la souche parentale.

En titrant les activités AC et Ptx dans les surnageants de culture des 3 inoculats, il apparaît que le clone VIIIA7 secrète environ 10 fois plus d'AC activable par la calmoduline que la souche parentale.

Le clone 7-1 ne secrète pas de quantité décelable de cet enzyme. La production de Ptx, estimée à partir des effets LPF dans la souris dans laquelle on a effectué une injection par voie intraveineuse et des effets d'agglomération CHO, est également environ 2 fois supérieure dans VIIIA7 que dans la souche parentale et n'est pas décelée dans le clone 7-1.

Il apparaît que l'alvéolite hémorragique léthale aiguë qui suit l'infection intranasale de la souris avec le variant VIIIA7, est due à une libération plus importante d'AC et de Ptx, agissant tous deux comme agents augmentant le cAMP intracellulaire.

Dans les expériences ci-après on a déterminé les effets inhibiteurs spécifiques des anticorps formés contre des antigènes de cellules entières, FHA, Ptx ou AC.

Comme le montre le tableau 2, l'épreuve respiratoire léthale avec le clone VIIIA7 est fortement reproductible et induit la mort des souris dans les 4 jours. Les survivants au 5ème jour sont totalement guéris.

En préincubant l'inoculum d'épreuve avec des sérums variés, on constate que seulement les anticorps anti-FHA et anti-AC sont protecteurs vis-à-vis de l'alvéolite hémorragique aiguë observée constamment dans les témoins non immunisés. Les anticorps anti-Ptx ne permettent pas de protéger la souris contre une épreuve expérimentale tout en possédant cependant les effets protecteurs contre des effets cytopathogènes sur des cellules en culture.

| | témoin | Sérum Normal | Vaccin anti-cellule entière | | Anti-FHA | | | Anti-Ptx | | Anti-AC (polyclonal) | | | Anti-AC (monclonal) | | | |
|---|---|---|---|---|---|---|---|---|---|---|---|---|---|---|---|---|
| $\log_{10}$ dilution du sérum | | -1 | -1 | -2 | -1 | -1,6 | -2 | -1 | -2 | -1 | -1,6 | -2,2 | -2,2 | -2,5 | -2,8 -3,1 | |
| Exp.1: $1,6 \times 10^9$ CFU/ml[*] (AC=76 u/ml)[+] | | | | | | | | | | | | | | | | |
| Léthalité [‡] | 14/15 | 4/5 | 4/5 | 4/5 | 0/5 | 0/5 | 3/5 | 4/5 | 5/5 | 0/5 | 0/5 | 3/5 | | | | |
| % inactivation d'AC [§] | 0 | 0 | 0 | 0 | 0 | 0 | 0 | 0 | 0 | 89 | 84,5 | 56 | | | | |
| % inhibition de Ptx [°] | 0 | 0 | 0 | 0 | 0 | 0 | 0 | 100 | 100 | 0 | 0 | 0 | | | | |
| Exp.2: $2,7 \times 10^9$ CFU/ml[*] (AC=84 u/ml)[+] | | | | | | | | | | | | | | | | |
| Léthalité [‡] | 9/10 | 5/5 | | | | | | 4/5 | 4/5 | 0/5 | 2/5 | 4/5 | 1/5 | | | |
| % inactivation d'AC [§] | 0 | 0 | | | | | | 0 | 0 | 78 | 67 | 52 | 0 | | | |
| % inhibition de Ptx [°] | 0 | 0 | | | | | | 100 | 100 | 0 | 0 | 0 | 0 | | | |
| Exp.3: $2,6 \times 10^9$ CFU/ml[*] (AC=98 u/ml)[+] | | | | | | | | | | | | | | | | |
| Léthalité [‡] | 5/5 | 4/5 | | | | | | | | | | | 1/5 | 0/5 | 0/5 | 1/5 |
| % inactivation d'AC [§] | 0 | 0 | | | | | | | | | | | 0 | 15 | 25 | 0 |
| % inhibition de Ptx [°] | 0 | 0 | | | | | | | | | | | 0 | 0 | 0 | 0 |

[*] - densité de l'inoculum
[+] - activité de l'AC dans le surnageant de l'inoculum
[‡] - nombre de souris mortes/total
[§] - inactivation in vitro de l'AC extracellulaire dans l'inoculum
[°] - inhibition des effets d'agglomération - CHO du surnageant de l'inoculum.

On considère que les effets protecteurs des anti-FHA sont dus à une inhibition efficace de l'adhérence bactérienne par ces anticorps, conduisant à une rapide disparition de B. pertussis de la surface de l'épithélium cible.

L'efficacité des anticorps polyclonaux anti-AC, inhibant l'activité catalytique de l'enzyme, suggère que l'AC de Bordetella pertussisintervient dans la pathogénèse de la coqueluche comme toxine majeure responsable pour le syndrome cytopathogène local dans le tractus respiratoire.

Les effets protecteurs de l'anticorps monoclonal 8-25 obtenu à partir de la souche d'hybridome I sont mis en évidence par analyse SDS-PAGE de surnageant concentré, d'extrait bactérien et de préparations d'AC purifiée et immunoblot à l'aide d'une fraction avec 18% de sulfate de sodium de l'ascite 8-25 dilué au 1/100 (35 mg de protéines totales/ml).

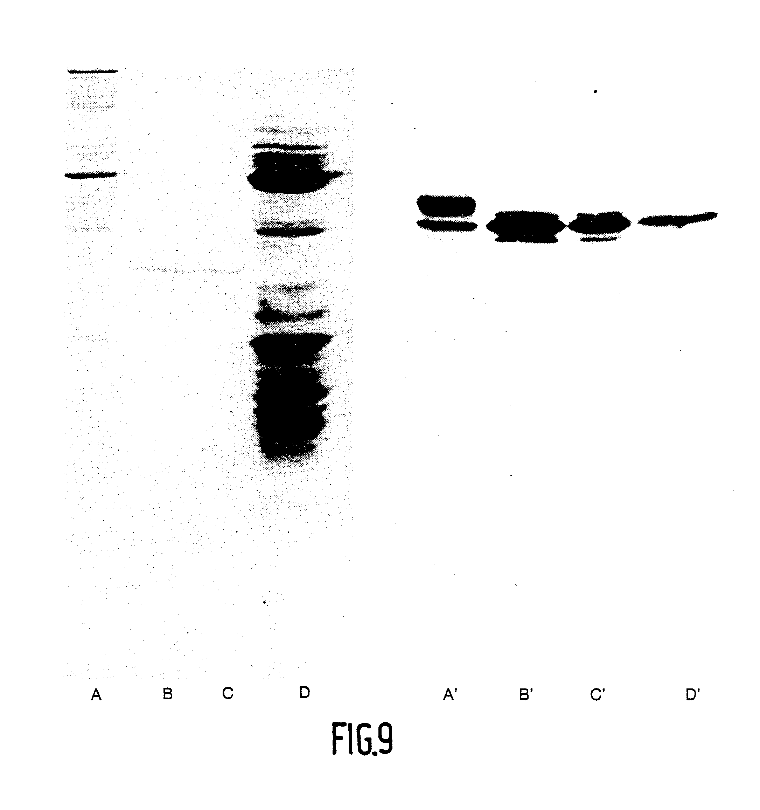

Les photos de l'analyse SDS-PAGE et de l'immunoblot sont rapportées sur les figures 9a et 9b respectivement.

Les pistes A, B, C et D sur la figure 9a correspondent respectivement à un surnageant concentré 115 fois (activité 9 267 u/ml), à de l'AC pure (1 590 u/ml), à une autre préparation d'AC pure (795 u ml) et à un extrait bactérien (<> 8,8 M) (231 u/ml). Les pistes A', B', C' et D' de la figure 9b correspondent aux produits des préparations ci-dessus transférées et immunoprécipitées une préparation d'IgG1 d'anticorps monoclonal 8-25 anti-AC.

L'examen de ces résultats montre que ces anticorps monoclonaux reconnaissent spécifiquement le composé de 43-45 kDa de l'AC de B. pertussis.

Ces expériences montrent que l'AC de B. pertussis est un facteur pathogène majeur et un antigène protecteur contre la coqueluche.

EXEMPLE 9 : Pouvoir immunoprotecteur de l'adényl cyclase de Bordetella pertussis.

On rapporte ci-après les résultats obtenus en étudiant les conditions d'induction d'une immunité spécifiquement protectrice contre les effets cytopathogènes de cette adényl cyclase :

1/ - METHODOLOGIE :

1.1. protocoles d'immunisation :

On injecte, par voie sous cutanée, à des souris Swiss ("outbred"Secific Pathogen-Free),femelles, agées de 3 semaines, des préparations immunogènes ou non.

L'adényl cyclase est purifiée sur Affi-Gel calmoduline (Biorad) selon la méthode de D. Ladant et al. J. Biol. Chem. 1986, 261:16264-16269. Les contrôles de pureté et de spécificité antigénique sont pratiqués par analyse électrophorétique et immunochimique.

Divers essais préliminaires montrant que la molécule administrée seule n'est pas immunogène, le mélange adényl cyclase + Affi-Gelcalmoduline est injecté.

Les suspensions bactériennes sont préparées par culture en milieu synthétique défini de Stainer et Scholte et ajustées aux concentrations choisies par mesure de la densité optique à 650 nm et du nombre d'unités formant colonie (CFU), sur milieu de Bordet-Gengou au sang.

Les suspensions bactériennes vaccinales sont "inactivées" par chauffage soit à 56° C ("vaccin classique") soit à 80° C (inactivation complète des effets immunomodulateurs de la pertussis-toxine).

La neutralisation de l'épitope adényl cyclase est réalisée en incubant la suspension bactérienne avec l'anticorps monoclonal 8-25 pendant 18 heures à 4° C.

1.2. Epreuve :

Instillation intranasale, à chaque souris, d'un inoculum bactérien préparé à partir d'un variant de la souche de B.pertussis 18323, hémolytique et producteur d'adényl cyclase (70-100 u ml, pour une suspension titrant 2 à 5 $\times$ $10^8$ CFU/ml), quinze jours après la dernière injection.

Les souris sont observées pendant vingt jours. Les analyses statistiques sont effectuées après établissement de la survie moyenne selon la conversion de Liddel (F.D.K.Liddel Microbiol. Rev. 1978, 42:237-249) avec thêta = 0,1 et T = 20.

## 2 - RESULTATS ET COMMENTAIRES :

Les résultats de l'expérience, figurant au tableau 3, montrent que B.pertussis, productrice d'adényl cyclase (BP AC+) induit une immunité protectrice contre l'épreuve infectieuse léthale et que la pré-incubation de cette suspension bactérienne immunogène avec l'anticorps monoclonal reconnaissant spécifiquement l'adényl cyclase réduit son pouvoir immunoprotecteur à des valeurs aussi faibles que celles conférées par le variant non producteur d'adényl cyclase (BP AC-).

Les résultats de l'expérience, figurant au tableau 4, montrent que l'injection d'adényl cyclase associée au gel d'agarose-calmoduline confère une protection aussi élevée que les bactéries exprimant cet antigène (BP 18323 et BP 8132) et significativement efficace par comparaison avec les résultats observés après injection du gel d'agarose-calmoduline seul ou d'une suspension de vaccin commercial (DTCP).

## TABLEAU 3

Pouvoir immunoprotecteur de B.pertussis 18323, productrice d'adényl cyclase (BP AC+) et abolition de ce pouvoir immunoprotecteur en préincubant la suspension immunogène avec l'anticorps monoclonal anti-adényl cyclase (anti-AC), étudié comparativement avec un variant isogènique non producteur de l'adényl cyclase (BP AC-) et diverses autres Bordetella.

| Immunogène* | n°survivantes | survie moyenne +-SD |
|---|---|---|
| BP AC+ | 7/12 | 0,635+-0,11** |
| BP AC+ +anti-AC | 4/17 | 0,37 +-0,09** |
| BP AC- | 4/11 | 0,46 +-0,11** |
| BPP63-2 | 6/12 | |
| BB530 | 4/12 | |
| BB552 | 5/12 | |
| BA | 5/12 | |
| non | 3/12 | 0,32 +-0,10** |

BPP = B.parapertussis

BB = B.bronchiseptica

BA = B.avium

* suspension chauffée 10 min. à 80° C (inhibition complète de l'effet immunostimulant de la pertussis toxine).

** protection hautement significative de BP AC+, $p < 10^{-5}$ (test $t$ de Student).

TABLEAU 4

Pouvoir immunoprotecteur de l'adényl cyclase purifiée de <u>B.pertussis</u> (AC), étudié comparativement avec divers vaccins "classiques".

| Immunogène | dose | n° injections | n° survivantes | survie moyenne +-SD |
|---|---|---|---|---|
| AC+CaM-Affigel | 5µg | 2 | 9/12 | 0,75 +-0,10* |
| " | " | 4 | 9/12 | 0,76 +-0,09 |
| CaM-Affigel | | 2 | 5/12 | 0,49 +-0,11* |
| " | | 4 | 4/12 | 0,45 +-0,10 |
| BP18323 | $2 \times 10^8$ CFU | 1 | 9/12 | 0,76 +-0,095 |
| BP8132 | " | 1 | 9/11 | 0,80 +-0,09 |
| DTCP lot M5342 | 1/5 ** | 1 | 4/12 | 0,445+-0,10 |

CaM-Affigel = Affi-Gel calmoduline "Biorad"

* protection hautement significative de AC+CaM-Affigel, $p < 10^{-4}$ (test $\underline{t}$ de Student).

** théoriquement équivalent à $2 \times 10^8$ CFU.

## Revendications

1. Préparations d'adényl cyclase ou AC, caractérisées en ce qu'elles possèdent une pureté élevée et sont pratiquement totalement dépourvues de produits contaminants bactériens, notamment de pertussis toxines, de lipopolysaccharide (ou LPS) et d'hémagglutinine filamenteuse (ou FHA).

2. Préparations selon la revendication 1, caractérisées en ce que l'AC se présente sous une forme homogène sédimentant sur un gradient de densité en saccharose avec un coefficient S égal à 3,6.

3. Préparations selon la revendication 1 ou 2, caractérisées en ce que l'AC existe sous deux formes moléculaires de 45 et 43 kDa respectivement, structurellement apparentées.

4. Préparations d'AC caractérisées en ce qu'elles possèdent une activité pouvant atteindre et même dépasser 1600 µmole de cAMP min' mg'.

5. Préparations selon l'une quelconque des revendications 1 à 4, caractérisées en ce que l'AC est libre ou sous forme d'un complexe, en association avec des protéines eucaryotes et plus spécialement avec la calmoduline.

6. Préparations selon la revendication 5, caractérisées par une constante d'association avec la calmoduline de 10 nM environ.

7. Préparations selon l'une quelconque des revendications 1 à 6, caractérisées en ce que l'activité catalytique est portée par un fragment de 25 kDa, site de combinaison à la calmoduline et protégé par celle-ci contre les digestions trypsiques.

8. Préparations selon l'une quelconque des revendications 1 à 6, caractérisées en ce qu'elles sont élaborées à partir de cultures de bactéries exprimant l'AC, plus spécialement de bactéries pathogènes dont l'AC est capable d'interférer avec l'AC des cellules eucaryotes.

9. Préparations selon la revendication 8, caractérisées en ce que les bactéries sont du genre Bordetella ou Bacillus anthracis.

10. Préparations selon la revendication 9, caractérisées en ce que l'AC provient de Bordetella responsables de pathologies respiratoires des vertébrés telles que chez l'homme, Bordetella pertussis et Bordetella parapertussis, et chez l'animal Bordetella bronchiseptica et Bordetella avium.

11. Procédé d'obtention de préparations d'AC de haute pureté, caractérisé par l'étape de mise en contact d'un surnageant préalablement concentré de cultures de bactéries exprimant l'adénylate cyiase, ou d'un extrait de ces bactéries avec de la calmoduline.

12. Procédé selon la revendication 11, caractérisé en ce que pour obtenir l'enzyme sous forme libre, on utilise de la calmoduline fixée à un support, puis on récupère l'enzyme adsorbée à l'aide d'un agent dénaturant qui est à son tour éliminé, et on recueille la préparation avec l'enzyme libre.

13. Procédé selon la revendication 12, caractérisé en ce que le support est constitué par un matériau inerte vis-à-vis de la préparation renfermant l'enzyme, et capable de retenir les molécules de poids moléculaire élevé, tel qu'un gel ou un matériau filtrant.

14. Procédé selon la revendication 13, caractérisé en ce que le matériau filtrant est en nitrocellulose ou en une matière plastique et présente une porosité de 0,45-0,22 μm.

15. Procédé selon la revendication 11, caractérisé en ce que l'agent dénaturant est de l'urée, de préférence 4 à 8,8 M.

16. Procédé selon la revendication 11, pour obtenir l'enzyme sous forme d'un complexe avec la calmoduline, caractérisé en ce qu'on ajoute de la calmoduline à une préparation enrichie en AC puis on met en contact le mélange, de préférence en réalisant une chromatographie d'affinité, avec un échangeur d'ions de faible porosité tel que le DEAE-Sephacel®.

17. Procédé selon la revendication 16, caractérisé en ce que la préparation enrichie en AC est obtenue à partir d'un surnageant de culture concentré, dont le pH a été amené à 7,0 et filtré à l'aide d'un échangeur d'ions, avantageusement du DEAE-Sephacel®.

18. Procédé selon l'une quelconque des revendications 11 à 17, caractérisé en ce que le surnageant concentré est obtenu en soumettant un surnageant de cultures bactériennes exprimant l'AC, à une ou plusieurs opérations de filtrations, à l'aide de filtres de nitrocellulose ou de matière plastique de porosité avantageusement de 0,45 à 0,22 μm, puis, en incubant les filtres avec un agent détergent afin de libérer l'AC et en éliminant de la préparation d'AC les matériaux insolubles présents.

19. Procédé selon la revendication 18, caractérisé en ce que l'agent détergent est du Triton® ou du NP40®.

20. Procédé selon l'une quelconque des revendications 11 à 18, caractérisé en ce que l'extrait bactérien est obtenu par traitement des cellules bactériennes exprimant l'adényl cyclase avec de l'urée et récupération du surnageant.

21. Procédé selon l'une quelconque des revendications 11 à 20, caractérisé en ce que les bactéries exprimant l'adényl cyclase utilisées pour les cultures sont choisies parmi les bactéries pathogènes dont l'AC est capable d'interférer avec l'AC des cellules eucaryotes, plus particulièrement de Bordetella ou Bacillus anthracis et parmi les Bordetella, plus spécialement, Bordetella pertussis, Bordetella bronchiseptica, Bordetella parapertussis et Bordetella avium.

22. Préparations d'anticorps polyclonaux formés contre les préparations enzymatiques purifiées selon l'une quelconque des revendications 1 à 20.

23. Anticorps monoclonaux tels qu'obtenus par fusion, à un myélome de souris, de lymphocytes spléniques de souris immunisées avec l'adényl cyclase purifiée.

24. Préparations d'immunoglobulines monoclonales caractérisées en ce qu'elles reconnaissent spécifiquement un épitope de l'AC purifiée qui permet l'induction de la synthèse d'anticorps protecteurs.

25. Préparations d'anticorps monoclonaux (8-25) qui immunoprécipite, dans les extraits bruts, et les préparations de l'enzyme purifiée à partir des cellules bactériennes, un triplet de 50,45 et 43 kDa ainsi qu'une protéine de haut poids moléculaire supérieur à 100 kDa.

26. Souche d'hybridome productrice d'anticorps monoclonaux selon la revendication 25, déposée à la Collection Nationale de Cultures de Microorganismes sous le n° I-610 le 9 Octobre 1986.

27. Application des anticorps selon les revendications 22 à 24, en tant que principe actif pour protéger l'homme ou l'animal des effets pathogènes des bactéries en association avec des excipients pharmacologiquement acceptables.

28. Application des anticorps selon les revendications 22 à 24 pour la purification de l'AC.

29. Application des anticorps selon les revendications 22 à 24 comme réactif de diagnostic.

30. Epitope actif de l'AC reconnu spécifiquement par les préparations d'anticorps monoclonaux selon la revendication 24 ou 25.

31. Vaccins caractérisés en ce qu'il s'agit de vaccins moléculaires renfermant un épitope actif de l'AC en association avec un véhicule pharmaceutique. capable d'induire une protection contre une infection et des effets toxiques chez l'homme ou l'animal.

FIG.1

FIG.2

FIG.3

4a

← 43 kDa

← 25 kDa →

0   2   4   8   12  20  30

min de digestion

4b

activité (%)

100

50

10   20   30 min.

FIG. 4

FIG.5

FIG. 6

0 272 174

FIG. 7

FIG.8

EDS - PAGE
a

IMMUNOBLOT
McAb 8-25

A     B     C     D        A'     B'     C'     D'

# FIG.9